# EUROPÄISCHE PATENTANMELDUNG

(11) **EP 0 958 844 A2**
(43) Veröffentlichungstag der Anmeldung: **24.11.1999**
(21) Anmeldenummer: 99108856.8
(22) Anmeldetag: 04.05.1999
(51) Int. Cl.: A61N 2/02

(54) **Magnetstimulationsgerät**

(30) Priorität: 15.05.1998 DE 19822019
(71) Anmelder: SIEMENS AKTIENGESELLSCHAFT, 80333 München (DE)
(72) Erfinder: Schweighofer, Peter,Dipl.-Ing., 90419 Nürnberg (DE); Schmitt, Franz, Dipl.-Phys., 91054 Erlangen (DE); Moritz, Michael, Dipl.-Ing., 95490 Mistelgau (DE)

(57) **Zusammenfassung**

Das erfindungsgemäße Magnetstimulationsgerät umfaßt wenigstens eine Stimulationsspule, die mit ihren Anschlüssen am Ausgang wenigstens eines steuerbaren Stromrichters angeschlossen ist und die eine Induktivität, Strom- und Spannungstragfähigkeit aufweist, so daß die von ihr erzeugten Stimulationspulse wenigstens ein Volumen in der Größenordnung von Gliedmaßen, Kopf oder Rumpf eines Patienten mit einer vorgebbaren magnetischen Feldstärke durchsetzen, wobei der steuerbare Stromrichter wenigstens einen ein- und abschaltbaren Leistungshalbleiterschalter mit kurzen Schaltzeiten aufweist, der Stromrichter mit seinem Eingang an einen Spannungszwischenkreis geschaltet ist und der Spannungszwischenkreis sowie der steuerbare Stromrichter eine Auslegung für hohe Ausgangsspannungen und -ströme aufweisen, so daß auch in tieferliegendem neuromuskularen Gewebe eines Patienten Aktionspotentiale auslösbar sind. Ein derartiges Magnetstimulationsgerät bietet größere Freiheiten bei der Wahl der Stimulationspulsformen.

## Beschreibung

Die Erfindung betrifft ein Magnetstimulationsgerät.

Magnetstimulationsgeräte dienen im Bereich der medizinischen Diagnostik und Therapie zur magnetischen Stimulation von Nervenfasern und muskularem Gewebe. Im Vergleich zur elektrischen Stimulation mittels Reizstrom liegt der Vorteil der Magnetpulsstimulation in der geringeren Schmerzhaftigkeit der Reizung, da bei der Magnetpulsstimulation keine höheren Stromdichten im Bereich der Schmerzrezeptoren der Haut auftreten. Ein weiterer Vorteil der Magnetstimulation liegt in dem höheren Penetrationsvermögen, wodurch auch die Erregung von tieferliegendem Gewebe, insbesondere tieferliegenden Nervenfasern, möglich ist.

Das Buch von R. F. Schmidt (Herausgeber) 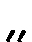 Neuro- und Sinnesphysiologie", Springer, zweite, korrigierte Auflage 1995, Kapitel 2 und 3, beinhaltet eine genau Beschreibung neurophysiologischer Vorgänge. Das Nervensystem beispielsweise koordiniert die Aktivitäten der verschiedenen Organe und Reaktionen des Körpers auf die Umwelt. Dies geschieht vornehmlich durch eine Änderungen des Potentials von Nervenzellen. Alle Zellen besitzen ein Ruhepotential. Beim Ruhepotential befinden sich alle Membranströme einer Zelle im Gleichgewicht. Wird das Membranpotential durch einen zusätzlichen Membranstrom, der z.B. durch einen äußeren Einfluß in die Zelle gelangt, depolarisiert, so geht dies mit einer Potentialänderung einher, einem sogenannten Aktionspotential. Vorgenannter depolarisierender Membranstrom wird auch Reiz genannt. Das Auslösepotential für ein Aktionspotential heißt Schwelle. An der Schwelle ändert sich das Gleichgewicht der Membranströme. Es treten für kurze Zeit zusätzliche Membranströme auf, die die Membran depolarisieren. Man nennt diesen Zustand auch Erregung. Mit einem Aktionspotential geht eine Aktion einher.

So wird z.B. jede Zuckung einer Muskelfaser durch ein Aktionspotential in der Muskelfaser begleitet und jede Reaktion einer Sinneszelle auf einen Sinnesreiz wird durch Aktionspotentiale weitergeleitet.

In der EP 0 182 160 A1 ist ein Gerät zur Erzeugung elektromagnetischer Pulse mit halbkreisförmiger Form beschrieben, welches insbesondere zur Förderung der Mikrozirkulation des Blutes im Bereich der Haarwurzeln und der Haut, beispielsweise gegen Haarausfall dient. Dazu ist an einem Wechselspannungstransformator eine Diodengleichrichterbrücke in Graetz-Schaltung angeschlossen, die eine pulserzeugende Spule speist .

In der DE 36 07 235 A1 ist ein Gerät zur Erzeugung von unipolaren Luftionen und elektromagnetischen Impulsfeldern zur Herabsetzung der menschlichen Reaktionszeit bei gleichzeitiger Erhöhung der Aufmerksamkeits-Bereitschaft bekannt. Zur Erzeugung der elektromagnetischen Impulsfelder ist an eine Spannungsquelle ein Frequenzgenerator mit nachgeschaltetem Auskoppelverstärker und eine das Impulsfeld erzeugende Spule angeschlossen.

In der DE 41 32 428 A1 ist ein Magneto-Therapiegerät zur magnetotherapeutischen Behandlung beschrieben. Zur Erzeugung eines pulsierenden Magnetfeldes ist an eine Batterie ein astabiler Multivibrator angeschlossen, der zwei mit Eisen gefüllte Zylinderspulen speist. Das Gerät ist als Taschengerät ausgebildet.

In der US-Patentschrift 5 743 844 ist ein Gerät zur Therapie mittels pulsierender elektromagnetischer Felder zur Förderung der Heilung von Knochen- und Körpergewebe, insbesondere in der Ausgestaltung als am Körper tragbares, batteriegespeistes Gerät bekannt. Dazu wird aus zwei Spannungsquellen unterschiedlicher Spannungshöhe über eine spezielle Schaltung, die als Kernelemente zwei Feldeffekttransistoren und zwei Kondensatoren beinhaltet, eine das Magnetfeld erzeugende Spule gespeist. Dabei besitzt vorgenannte Schaltung ein festes Puls-Pausen-Zeitverhältnis.

Die in den vorausgehend zitierten Patentschriften beschriebenen Geräte sind alle derart konzipiert, daß die von ihnen erzeugten magnetischen Puls- bzw. Wechselfelder unterhalb der Schwelle zur Auslösung von Aktionspotentialen auf den menschlichen Körper wirken. Die damit tatsächlich erreichbaren Wirkungen im menschlichen Körper sind teilweise sehr diffus und wissenschaftlich umstritten. Bei Magnetstimulationsgeräten, die gezielt Aktionspotentiale, insbesondere in tieferliegendem neuromuskularen Gewebe auslösen, handelt es sich um eine ganz andere Kategorie von Geräten. Nicht nur Einsatz und therapeutische Wirkung dieser Geräte ist anders, sondern auch die dafür aufzubringenden elektrischen Leistungen sind um ein Vielfaches größer, was sich in entsprechend hohen Strom- und Spannungswerten niederschlägt. Dazu sind die in den vorausgehend zitierten Patentschriften beschriebenen Geräte aufgrund ihrer gesamten niederspannungs- und kleinstromtechnischen Auslegung nicht geeignet.

Ein Magnetstimulationsgerät zur Auslösung von Aktionspotentialen, auch in tieferliegendem neuromuskularen Gewebe ist im Aufsatz von M. Schmid, T. Weyh und B.-U. Meyer "Entwicklung, Optimierung und Erprobung neuer Geräte für die magnetomotorische Stimulation von Nervenfasern", Biomedizinische Technik 38 (1993), Seiten 317 bis 324, beschrieben. Es weist eine Stimulationsspule auf, die zusammen mit einem Hochspannungskondensator einen Parallelschwingkreis bildet, also als resonante Schaltung arbeitet. Der Hochspannungskondensator wird von einem regelbaren Netzteil aufgeladen und akkumuliert dadurch die für die Abgabe eines Stimulationspulses erforderliche Pulsenergie. Das regelbare Netzteil ist hierzu mit den Anschlüssen seiner Ladeschaltung an den Hochspannungskondensator geführt.

In den Stromkreis des Hochspannungskondensators, der sich über die Stimulationsspule schließt, ist als elektronischer Schalter ein Thyristor eingefügt. Während der Hochspannungskondensator zur Vorbereitung der Pulsabgabe vom regelbaren Netzteil geladen wird, bleibt der Thyristor geöffnet, der Parallelschwingkreis ist unterbrochen. Hat die Ladespannung den vom Anwender gewünschten Wert erreicht, trennt ein zur Ladeschaltung gehörender Ladeschalter den Hochspannungskondensator vom regelbaren Netzteil und der Stimulationspuls wird durch Zünden des Thyristors ausgelöst. Der Hochspannungskondensator entlädt sich über die Stimulationsspule. Ist der Hochspannungskondensator vollständig entladen, kehrt sich die Richtung des Energieflusses um. Ein jetzt mögliches Aufladen des Hochspannungskondensators mit umgekehrter Polarität wird dadurch vermieden, daß parallel zum Hochspannungskondensator ein Freilaufzweig als zusätzlicher Strompfad angeordnet ist.

Der Freilaufzweig umfaßt eine Freilaufdiode und einen dazu in Reihe geschalteten Freilaufwiderstand.

In dem Freilaufzweig wird die von der Stimulationsspule zurückschwingende Energie größtenteils aufgezehrt. Die Freilaufdiode sorgt dafür, daß der Freilaufzweig erst dann den Strom übernimmt, wenn die Rückspeisung aus der Stimulationsspule den Entladevorgang des Hochspannungskondensators ablöst (Kommutierung). Schließlich gibt auch der bei umgekehrter Polarität geringfügig aufgeladene Hochspannungskondensator seine Energie an den Freilaufwiderstand ab.

Die Resonanzfrequenz des von der Stimulationsspule und dem Hochspannungskondensator gebildeten Parallelschwingkreises ist durch die Wahl der Kapazität des Hochspannungskondensators und der Induktivität der Stimulationsspule festgelegt und liegt im Bereich von 1 bis 3 kHz. Variiert man die Kapazität des Hochspannungskondensators, dann läßt sich die Resonanzfrequenz des Parallelschwingkreises und damit die Geschwindigkeit des Stromanstiegs in der Stimulationsspule verändern. Die Reizintensität wird durch die initiale Spannung am Hochspannungskondensator festgelegt. Als weiterer Parameter ist nur noch die Repetitionsrate einstellbar, die im Bereich um 10 Hz liegt.

Weiterhin ist aus der DE 196 07 704 A1 eine Vorrichtung zur magnetischen Anregung von neuromuskularem Gewebe bekannt. Die bekannte Vorrichtung weist eine Anregungsspule (Stimulationsspule) auf, die zusammen mit einem Speicherkondensator (Hochspannungskondensator) einen Parallelschwingkreis bildet, also ebenfalls als resonante Schaltung arbeitet. Auch bei dieser Vorrichtung lassen sich nur Resonanzfrequenzen im Bereich von 1 bis 3 kHz realisieren.

Aufgabe der vorliegenden Erfindung ist es deshalb, ein Magnetstimulationsgerät zur Auslösung von Aktionspotentialen, auch in tieferliegendem neuromuskularen Gewebe zu schaffen, das größere Freiheiten bei der Wahl der Stimulationspulsformen bietet.

Diese Aufgabe wird erfindungsgemäß durch die Merkmale im Anspruch 1 gelöst. Vorteilhafte Ausgestaltungen der Erfindung sind jeweils Gegenstand von weiteren Ansprüchen.

Das erfindungsgemäße Magnetstimulationsgerät umfaßt wenigstens eine Stimulationsspule, die mit ihren Anschlüssen am Ausgang wenigstens eines steuerbaren Stromrichters angeschlossen ist und die eine Induktivität, Strom- und Spannungstragfähigkeit aufweist, so daß die von ihr erzeugten Stimulationspulse wenigstens ein Volumen in der Größenordnung von Gliedmaßen, Kopf oder Rumpf eines Patienten mit einer vorgebbaren magnetischen Feldstärke durchsetzen, wobei der steuerbare Stromrichter wenigstens einen ein- und abschaltbaren Leistungshalbleiterschalter mit kurzen Schaltzeiten aufweist, der steuerbare Stromrichter mit seinem Eingang an einen Spannungszwischenkreis geschaltet ist und der Spannungszwischenkreis sowie der steuerbare Stromrichter eine Auslegung für hohe Ausgangsspannungen und -ströme aufweisen, so daß auch in tieferliegendem neuromuskularen Gewebe eines Patienten Aktionspotentiale auslösbar sind.

Unter dem Begriff "kurze Schaltzeit" sind bei dem erfindungsgemäßen Magnetstimulationsgerät Schaltzeiten von ca. 1 µs oder kleiner zu verstehen.

Bei dem Magnetstimulationsgerät nach Anspruch 1 wird die Stimulationsspule über einen Spannungszwischenkreis und einen steuerbaren Stromrichter mit der erforderlichen Pulsenergie versorgt. Das erfindungsgemäße Magnetstimulationsgerät arbeitet also nicht als resonante Schaltung, bei der die Stimulationsspule mit einem Hochspannungskondensator einen Parallelschwingkreis bildet.

Durch den Einsatz von ein- und abschaltbaren Leistungshalbleiterschaltern lassen sich größere Freiheiten in der Wahl der Stimulationspuisformen erzielen, da auf einen resonanten Betrieb verzichtet werden kann. Regelbare Netzteile mit speziellen Ladeschaltungen können entfallen.

Bei dem erfindungsgemäßen Magnetstimulationsgerät wird der Zwischenkreiskondensator des Spannungszwischenkreises mittels eines Netzteils aufgeladen. Das Netzteil muß nicht regelbar sein und ist vorzugsweise als Hochspannungsnetzteil ausgeführt. Spezielle Ladeschalter entfallen dann. Der Zwischenkreiskondensator wird im Gegensatz zum Hochspannungskondensator der bekannten Magnetstimulationsgeräte nicht mehr vollständig entladen und bestimmt auch nicht mehr irgendwelche Zeitverläufe bei den Stimulationspulsen. Der Zwischenkreiskondensator kann deshalb mit wesentlich höherer Kapazität ausgeführt werden. Die Formen der von der Stimulationsspule erzeugten Stimulationspulse werden lediglich über die Repetitionsrate und die Pulsdauer der Einschaltphasen der ein- und abschaltbaren Leistungshalbleiterschalter gesteuert.

Da die Stimulationsspule - im Gegensatz zu vergleichbaren Magnetstimulationsgeräten gemäß dem Stand der Technik - nun nicht mehr Teil eines Parallelschwingkreises ist, ergeben sich weitere Freiheitsgrade durch die Wahl der Induktivität der Stimulationsspule.

Im Rahmen der Erfindung kann der steuerbare Stromrichter als unipolarer Stromrichter, vorzugsweise als Chopper (Anspruch 2) oder als bipolarer Stromrichter, vorzugsweise als Wechselrichter (Anspruch 3), ausgeführt sein.

Die ein- und abschaltbaren Leistungshalbleiterschalter müssen kurze Schaltzeiten aufweisen, so daß hierfür derzeit vorzugsweise Transistoren, insbesondere IGBTs oder MOSFETs, Verwendung finden.

Die Erfindung sowie weitere vorteilhafte Ausgestaltungen, die Gegenstand der weiteren Ansprüche sind, werden im folgenden anhand von zwei Ausführungsbeispielen näher erläutert. Es zeigen:
- FIG 1: ein Magnetstimulationsgerät gemäß dem Stand der Technik,
- FIG 2: eine erste Ausführungsform eines Magnetstimulationsgerätes gemäß der Erfindung,
- FIG 3: eine zweite Ausführungsform eines Magnetstimulationsgerätes gemäß der Erfindung.

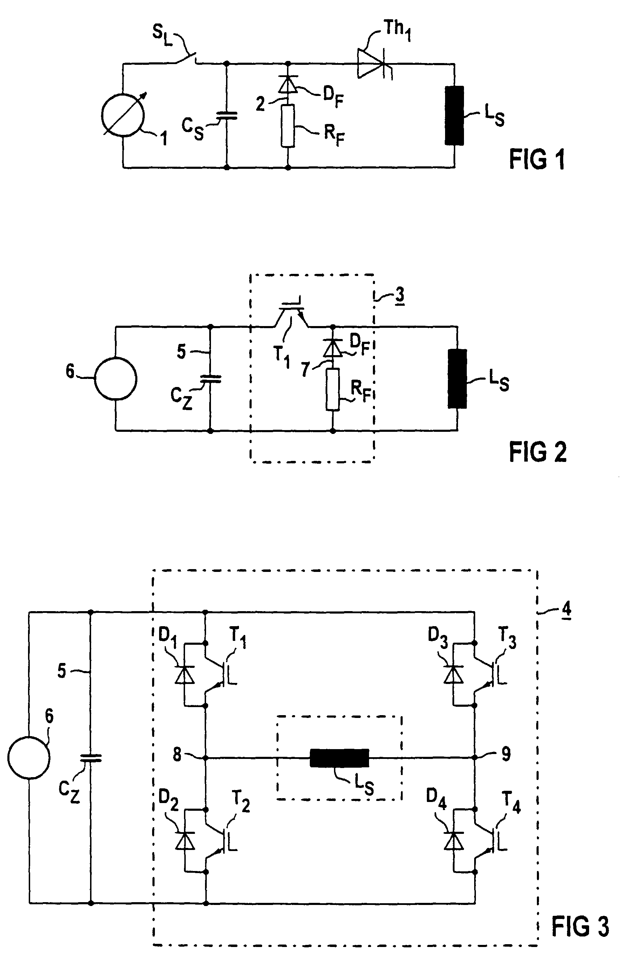

In FIG 1 ist mit L_{S} eine Stimulationsspule bezeichnet, die zusammen mit einem Hochspannungskondensator C_{S} einen Parallelschwingkreis bildet. Der Hochspannungskondensator C_{S} wird von einem regelbaren Netzteil 1 aufgeladen und akkumuliert dadurch die für die Abgabe eines Stimulationspulses erforderliche Pulsenergie. Das regelbare Netzteil 1 ist hierzu mit den Anschlüssen seiner Ladeschaltung an den Hochspannungskondensator C_{S} geführt. Von der Ladeschaltung des regelbaren Netzteils 1 ist nur der Ladeschalter S_{L} dargestellt.

In den Stromkreis des Hochspannungskondensators C_{S}, der sich über die Stimulationsspule L_{S} schließt, ist als elektronischer Schalter ein Thyristor Th₁ eingefügt. Während der Hochspannungskondensator C_{S} zur Vorbereitung der Pulsabgabe vom regelbaren Netzteil 1 geladen wird, bleibt der Thyristor Th₁ geöffnet, der Parallelschwingkreis ist unterbrochen. Hat die Ladespannung den vom Anwender gewünschten Wert erreicht, trennt der Ladeschalter S_{L} den Hochspannungskondensator C_{S} vom regelbaren Netzteil 1 und der Stimulationspuls wird durch Zünden des Thyristors Th₁ ausgelöst. Der Hochspannungskondensator C_{S} entlädt sich über die Stimulationsspule L_{S}. Ist der Hochspannungskondensator C_{S} vollständig entladen, kehrt sich die Richtung des Energieflusses um. Ein jetzt mögliches Aufladen des Hochspannungskondensators C_{S} mit umgekehrter Polarität wird dadurch vermieden, daß parallel zum Hochspannungskondensator C_{S} ein Freilaufzweig 2 als zusätzlicher Strompfad angeordnet ist.

Der Freilaufzweig 2 umfaßt eine Freilaufdiode D_{F} und einen dazu in Reihe geschalteten Freilaufwiderstand R_{F}.

In dem Freilaufzweig 2 wird die von der Stimulationsspule L_{S} zurückschwingende Energie größtenteils aufgezehrt. Die Freilaufdiode D_{F} sorgt dafür, daß der Freilaufzweig 2 erst dann den Strom übernimmt, wenn die Rückspeisung aus der Stimulationsspule L_{S} den Entladevorgang des Hochspannungskondensators C_{S} ablöst (Kommutierung). Schließlich gibt auch der bei umgekehrter Polarität geringfügig aufgeladene Hochspannungskondensator C_{S} seine Energie an den Freilaufwiderstand R_{F} ab.

Bei dem in FIG 1 gezeigten Magnetstimulationsgerät gemäß dem Stand der Technik ist die Resonanzfrequenz des von der Stimulationsspule L_{S} und dem Hochspannungskondensator C_{S} gebildeten Parallelschwingkreises durch die Wahl der Kapazität des Hochspannungskondensators C_{S} und der Induktivität der Stimulationsspule L_{S} festgelegt und liegt im Bereich von 1 bis 3 kHz. Variiert man die Kapazität des Hochspannungskondesators C_{S}, dann läßt sich die Resonanzfrequenz des Parallelschwingkreises und damit die Geschwindigkeit des Stromanstiegs in der Stimulationsspule L_{S} verändern. Die Reizintensität wird durch die initiale Spannung am Hochspannungskondensator C_{S} festgelegt. Als weiterer Parameter ist nur noch die Repetitionsrate einstellbar, die im Bereich um 10 Hz liegt.

Das erfindungsgemäße Magnetstimulationsgerät, von dem in FIG 2 und 3 jeweils ein Ausführungsbeispiel gezeigt ist, umfaßt wenigstens eine Stimulationsspule L_{S}, die mit ihren Anschlüssen am Ausgang wenigstens eines steuerbaren Stromrichters 3 (FIG 2) bzw. eines steuerbaren Stromrichters 4 (FIG 3) angeschlossen ist.

Im Rahmen der Erfindung kann der steuerbare Stromrichter als unipolarer Stromrichter (Stromrichter 3 in FIG 2) oder als bipolarer Stromrichter (Stromrichter 4 in FIG 3) ausgeführt sein.

Die Stromrichter 3 und 4 weisen jeweils wenigstens einen ein- und abschaltbaren Leistungshalbleiterschalter T₁ mit kurzen Schaltzeiten auf. Gemäß einer vorteilhaften Ausgestaltung der Erfindung sind die ein- und abschaltbaren Leistungshalbleiterschalter als Transistoren ausgeführt. Der Stromrichter 3 bzw. 4 ist mit seinem Eingang an einen Spannungszwischenkreis 5 geschaltet. Der Spannungszwischenkreis 5 weist wenigstens einen Zwischenkreiskondensator C_{Z} auf. Der Zwischenkreiskondensator C_{Z} wird von einem Netzteil 6, das nicht regelbar sein muß und das vorzugsweise als Hochspannungsnetzteil ausgeführt ist, aufgeladen.

Bei dem in FIG 2 gezeigten Magnetstimulationsgerät wird die Stimulationsspule L_{S} nach dem Einschalten des Transistors T₁ mit der im Zwischenkreiskondensator C_{Z} gespeicherten Energie versorgt und damit ein unipolarer Stimulationspuls ausgelöst. Nach dem Abschalten des Transistors T₁ wird der in der Stimulationsspule L_{S} fließende Strom über einen im unipolaren Stromrichter 3 angeordneten Freilaufzweig 7 abgebaut. Im Freilaufzweig 7 ist wenigstens eine Freilaufdiode D_{F} angeordnet. Im dargestellten Ausführungsbeispiel ist im Freilaufzweig 7 ein Freilaufwiderstand R_{F} in Reihe zur Freilaufdiode D_{F} geschaltet. Der Freilaufwiderstand R_{F} bestimmt die Zeitkonstante und kann auch entfallen, falls die parasitären Widerstände im Stromkreis ausreichend groß sind.

Bei dem in FIG 3 dargestellten Magnetstimulationsgerät ist der Stromrichter 4 als Vollbrücke und damit als bipolarer Stromrichter ausgeführt, umfaßt also zwei Brückenzweige. Der erste Brückenzweig wird von den Transistoren T₁ und T₂ und der zweite Brückenzweig von den Transistoren T₃ und T₄ gebildet. Die Stimulationsspule L_{S} ist mit ihrem ersten Anschluß an dem Mittenanzapfpunkt 8 des ersten Brückenzweiges und mit ihrem zweiten Anschluß an dem Mittenanzapfpunkt 9 des zweiten Brückenzweiges angeschlossen. Hinsichtlich des Zwischenkreises 5 mit seinem Zwischenkreiskondensator C_{Z} gelten die Ausführungen zu dem in FIG 2 dargestellten Magnetstimulationsgerät.

Den Transistoren T₁ bis T₄ ist jeweils eine Freilaufdiode D₁ bis D₄ parallel geschaltet. Die Freilaufdioden D₁ bis D₄ können entweder als separate Dioden ausgeführt sein oder sie sind als parasitäre Dioden Teil der betreffenden Transistoren T₁ bis T₄.

Der bipolare Stromrichter 4 erzeugt durch Einschalten der Transistoren T₁ und T₄ positive Ausgangsspannungen. Negative Ausgangsspannungen werden durch Einschalten der Transistoren T₂ und T₃ erzeugt.

Spezielle Ladeschalter S_{L}, die bei dem Magnetstimulationsgerät gemäß dem Stand der Technik (FIG 1) erforderlich sind, entfallen bei dem erfindungsgemäßen Magnetstimulationsgerät. Der Zwischenkreiskondensator C_{Z} wird im Gegensatz zum Hochspannungskondensator C_{S} (FIG 1) nicht mehr vollständig entladen und bestimmt auch nicht mehr irgendwelche Zeitverläufe bei den Stimulationspulsen. Der Zwischenkreiskondensator C_{Z} kann deshalb mit wesentlich höherer Kapazität ausgeführt werden. Die Formen der von der Stimulationsspule L_{S} erzeugten Stimulationspulse werden lediglich über die Repetitionsrate und die Pulsdauer der Einschaltphasen des Transistors T₁ (FIG 2) bzw. der Transistoren T₁ bis T₄ (FIG 3) gesteuert.

Da die Stimulationsspule L_{S} - im Gegensatz zu dem in FIG 1 dargestellten Magnetstimulationsgerät - nun nicht mehr Teil eines Parallelschwingkreises ist, ergeben sich bei dem erfindungsgemäßen Magnetstimulationsgerät weitere Freiheitsgrade durch die Wahl der Induktivität der Stimulationsspule L_{S}.

Zum Erzielen der notwendigen Schalterbelastbarkeit können die hier als einzelne Bauteile dargestellten ein- und abschaltbaren Leistungshalbleiterschaltelemente auch als Parallel- und/oder Reihenschaltungen von mehreren Transistoren und/oder IGBTs und/oder MOSFETs ausgeführt sein.

## Patentansprüche

1. Magnetstimulationsgerät, das folgende Merkmale umfaßt:
- Das Gerät beinhaltet wenigstens eine Stimulationsspule, die eine Induktivität, Strom- und Spannungstragfähigkeit aufweist, so daß die von ihr erzeugten Stimulationspulse wenigstens ein Volumen in der Größenordnung von Gliedmaßen, Kopf oder Rumpf eines Patienten mit einer vorgebbaren magnetischen Feldstärke durchsetzen.
- Die Stimulationsspule ist mit ihren Anschlüssen am Ausgang wenigstens eines steuerbarer Stromrichters angeschlossen, der wenigstens einen ein- und abschaltbaren Leistungshalbleiterschalter mit kurzen Schaltzeiten beinhaltet.
- Der steuerbare Stromrichter ist mit seinem Eingang an einen Spannungszwischenkreis geschaltet.
- Der Spannungszwischenkreis und der steuerbare Stromrichter weisen eine Auslegung für hohe Ausgangsspannungen und - ströme auf, so daß auch in tieferliegendem neuromuskularen Gewebe eines Patienten Aktionspotentiale auslösbar sind.

2. Magnetstimulationsgerät nach Anspruch 1, mit folgendem Merkmal:
- Der steuerbare Stromrichter ist als unipolarer Stromrichter, vorzugsweise als Chopper, ausgeführt.

3. Magnetstimulationsgerät nach Anspruch 1, mit folgendem Merkmal:
- Der steuerbare Stromrichter ist als bipolarer Stromrichter, vorzugsweise als Wechselrichter, ausgeführt.

4. Magnetstimulationsgerät nach Anspruch 3, mit folgendem Merkmal:
- Der Wechselrichter ist als Vollbrücke ausgebildet.

5. Magnetstimulationsgerät nach Anspruch 3, mit folgendem Merkmal:
- Der Wechselrichter ist als Halbbrücke ausgebildet.

6. Magnetstimulationsgerät nach Anspruch 1, mit folgendem Merkmal:
- Der ein- und abschaltbare Leistungshalbleiterschalter ist als Transistor ausgebildet.

7. Magnetstimulationsgerät nach Anspruch 6, mit folgendem Merkmal:
- Der ein- und abschaltbare Leistungshalbleiterschalter ist als IGBT ausgebildet.

8. Magnetstimulationsgerät nach Anspruch 6, mit folgendem Merkmal:
- Der ein- und abschaltbare Leistungshalbleiterschalter ist als MOS-FET ausgebildet.
